(19) Europäisches Patentamt
European Patent Office
Office européen des brevets

(11) Veröffentlichungsnummer : **0 044 482**
**B1**

(12) # EUROPÄISCHE PATENTSCHRIFT

(45) Veröffentlichungstag der Patentschrift : **16.11.83**

(21) Anmeldenummer : **81105388.3**

(22) Anmeldetag : **10.07.81**

(51) Int. Cl.$^3$ : **C 07 C103/365**, C 07 C103/375, C 07 C103/58, C 07 C103/737, C 07 C103/76, C 07 C143/68, C 07 C149/23, C 07 D249/08, C 07 D231/12, C 07 D307/68, A 01 N 37/22

(54) **N-Allenylalkyl-acetanilide, Verfahren zu ihrer Herstellung sowie ihre Verwendung als Fungizide.**

(30) Priorität : **22.07.80 DE 3027758**

(43) Veröffentlichungstag der Anmeldung : **27.01.82 Patentblatt 82/04**

(45) Bekanntmachung des Hinweises auf die Patenterteilung : **16.11.83 Patentblatt 83/46**

(84) Benannte Vertragsstaaten : **BE CH DE FR GB IT LI NL**

(56) Entgegenhaltungen :
**EP A 0 005 591**
**EP A 0 018 509**
**EP A 0 020 859**
**GB A 2 068 364**

(73) Patentinhaber : **BAYER AG**
**Zentralbereich Patente, Marken und Lizenzen**
**D-5090 Leverkusen 1, Bayerwerk (DE)**

(72) Erfinder : **Lunkenheimer, Winfried, Dr.**
**Bismarckstrasse 89**
**D-5600 Wuppertal 1 (DE)**
Erfinder : **Führer, Wolfgang, Dr.**
**Wehrstrasse 28**
**D-5202 Hennef 1 (DE)**
Erfinder : **Stetter, Jörg, Dr.**
**Gellertweg 4**
**D-5600 Wuppertal 1 (DE)**
Erfinder : **Brandes, Wilhelm, Dr.**
**Eichendorffstrasse 3**
**D-5653 Leichlingen 1 (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

N-Allenylalkyl-acetanilide, Verfahren zu ihrer Herstellung sowie ihre Verwendung als Fungizide

Die vorliegende Erfindung betrifft neue N-Allenylalkylacetanilide, ein Verfahren zu ihrer Herstellung und ihre Verwendung als Fungizide.

Es ist bereits bekannt geworden, daß N-Chloracetyl-N-(2,6-dialkylphenyl)-alanin-alkylester, wie beispielsweise N-Chloracetyl-N-(2,6-dimethylphenyl)-alaninethylester, mit gutem Erfolg zur Bekämpfung von pilzlichen Pflanzenkrankheiten eingesetzt werden können (vergleiche DE-OS 23 50 944). Deren Wirkung ist jedoch, insbesondere bei niedrigen Aufwandmengen und -konzentrationen, insbesondere auch bei der Bekämpfung von Phytophthora-Arten, nicht immer ganz befriedigend.

Es wurden neue N-Allenylalkyl-acetanilide der allgemeinen Formel

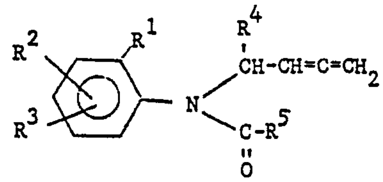

(I)

in welcher

$R^1$ für Wassertoff, Alkyl mit 1 bis 4 Kohlenstoffatomen oder Halogen steht,

$R^2$ für Wasserstoff oder Alkyl mit 1 bis 4 Kohlenstoffatomen steht,

$R^3$ für Wasserstoff oder Alkyl mit 1 bis 4 Kohlenstoffatomen steht,

$R^4$ für Wasserstoff oder Alkyl mit 1 bis 4 Kohlenstoffatomen steht,

$R^5$ für Furyl, Tetrahydrofuryl, Thiophenyl, Tetrahydrothiophenyl ; gegebenenfalls durch Methyl oder Ethyl substituiertes Isoxazolyl ; gegebenenfalls durch Cyano oder Thiocyanato substituiertes Alkyl mit 1 bis 4 Kohlenstoffatomen, Alkenyl und Alkinyl mit jeweils 2 bis 4 Kohlenstoffatomen ; gegebenenfalls substituiertes Cycloalkyl mit 3 bis 7 Kohlenstoffatomen oder Cycloalkenyl mit 4 bis 7 Kohlenstoffatomen, wobei als Substituenten genannt seien : Alkyl mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, Halogenalkenyl mit 2 bis 4 Kohlenstoff- und bis zu 5 gleichen oder verschiedenen Halogenatomen, Halogen, Alkoxy mit 1 bis 4 Kohlenstoffatomen sowie eine zwei- bis fünfgliedrige Methylenbrücke ; ferner für Halogenalkyl mit 1 oder 2 Kohlenstoffatomen und 1 oder 2 Halogenatomen ; sowie die Gruppierungen $-CH_2-Az$, $-CH_2-OR^6$ ; $-CH_2-SR^6$ ; $-OR^6$ ; $-SR^6$ ; $-CH_2-OSO_2R^6$, $-COOR^6$, $-CH_2-Ar$ und

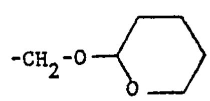

steht, wobei,

$R^6$ für gegebenenfalls durch Halogen, Cyano und Thiocyanato substituiertes Alkyl mit 1 bis 4 Kohlenstoffatomen, Alkenyl mit 2 bis 4 Kohlenstoffatomen, Alkinyl mit 2 bis 4 Kohlenstoffatomen und Alkoxyalkyl mit 1 bis 4 Kohlenstoffatomen in jedem Alkylteil steht,

Az für Pyrazol-1-yl, 1,2,4-Triazol-1-yl und Imidazol-1-yl steht, und

Ar für gegebenenfalls substituiertes Phenyl steht, wobei als Substituenten genannt seien : Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, Nitro, Cyano sowie gegebenenfalls durch Halogen und Alkyl mit 1 bis 4 Kohlenstoffatomen substituiertes Phenyl

sowie, wenn $R^5$ für $-CH_2Az$ steht, deren physiologisch verträglichen Säureadditions-Salze und Metallsalz-Komplexe gefunden.

Weiterhin wurde gefunden, daß man die neuen N-Allenyl-alkyl-acetanilide der Formel (I) erhält, wenn man N-Propargyl-acetanilide der Formel

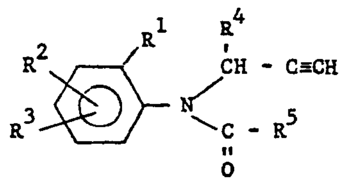

(II)

in welcher

$R^1$ bis $R^5$ die oben angegebene Bedeutung haben, in Gegenwart eines Katalysators mit Formaldehyd und Diisopropylamin umsetzt.

An die so erhaltenen Verbindungen der Formel (I) kann, wenn $R^5$ für $-CH_2-AZ$ steht, eine Säure oder ein Metallsalz addiert werden.

Die neuen Allenylalkyl-acetanilide weisen starke fungizide Eigenschaften auf. Dabei zeigen überraschenderweise die erfindungsgemäßen Verbindungen eine erheblich höhere Wirkung als der aus dem Stand der Technik bekannte N-Chloracetyl-N-(2,6-dimethylphenyl)-alanin-ethylester, welcher chemisch und wirkungsmäßig eine naheliegende Verbindung ist. Die erfindungsgemäßen Stoffe stellen somit eine Bereicherung der Technik dar.

Die erfindungsgemäßen N-Allenylalkyl-acetanilide sind durch die Formel (I) allgemein definiert. In dieser Formel stehen $R^1$, $R^2$, $R^3$ und $R^4$ vorzugsweise für Wasserstoff und geradkettiges oder verzweigtes Alkyl mit 1 bis 4 Kohlenstoffatomen. $R^1$ steht außerdem vorzugsweise für Halogen, wie insbesondere Fluor, Chlor oder Brom. $R^5$ steht vorzugsweise für Furyl, Tetrahydrofuryl, Thiophenyl, Tetrahydrothiophenyl, für gegebenenfalls durch Methyl oder Ethyl substituiertes Isoxazolyl sowie für gegebenenfalls durch Cyano oder Thiocyanato substituiertes Alkyl mit 1 bis 4 und Alkenyl sowie Alkinyl mit jeweils 2 bis 4 Kohlenstoffatomen ; für gegebenenfalls substituiertes Cycloalkyl mit 3 bis 7 Kohlenstoffatomen oder Cycloalkenyl mit 4 bis 7 Kohlenstoffatomen, wobei als Substituenten vorzugsweise infrage kommen : Alkyl mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, wie insbesondere Fluor-, Chlor- oder Bromatomen, Halogenalkenyl mit 2 bis 4 Kohlenstoff- und bis zu 5 gleichen oder verschiedenen Halogenatomen, wie insbesondere Fluor-, Chlor- und Bromatomen, Halogen, Alkoxy mit 1 bis 4 Kohlenstoffatomen sowie eine zwei- bis fünfgliedrige Methylenbrücke ; ferner vorzugsweise für Halogenalkyl mit 1 bis 2 Kohlenstoffatomen und 1 bis 2 Halogenatomen, wobei als Halogenatome vorzugsweise Fluor, Chlor und Brom genannt seien, sowie für die Gruppierungen $-CH_2-Az$, $-CH_2-OR^6$, $-CH_2-SR^6$, $-OR^6$, $-SR^6$, $-CH_2-OSO_2-R^6$, $-COOR^6$, $-CH_2Ar$ und

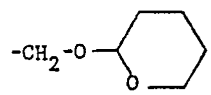

Az steht dabei vorzugsweise für Pyrazol-1-yl, 1,2,4-Triazol-1-yl und Imidazol-1-yl. $R^6$ steht vorzugsweise für gegebenenfalls durch Halogen, insbesondere Fluor, Chlor und Brom, Cyano und Thiocyanato substituiertes Alkyl mit 1 bis 4 und Alkenyl sowie Alkinyl mit jeweils 2 bis 4 Kohlenstoffatomen und für Alkoxyalkyl mit 1 bis 4 Kohlenstoffatomen in jedem Alkylteil. Ar steht vorzugsweise für gegebenenfalls substituiertes Phenyl, wobei als Substituenten vorzugsweise genannt seien : Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie Fluor- und Chloratomen, Nitro, Cyano sowie gegebenenfalls durch Halogen und Alkyl mit 1 bis 4 Kohlenstoffatomen substituiertes Phenyl.

Ganz besonders bevorzugt sind diejenigen substituierten N-Allenylalkyl-acetanilide der Formel (I), in denen $R^1$, $R^2$ und $R^3$ für Wasserstoff, Methyl, Ethyl, Isopropyl, sek.-Butyl und tert.-Butyl stehen ; $R^1$ außerdem für Chlor oder Brom steht ; $R^4$ für Wasserstoff, Methyl oder Ethyl steht ; und $R^5$ für 2-Furyl, 2-Thienyl, 2-Tetrahydrofuryl, 5-Methylisoxazol-3-yl, Methoxymethyl, Ethoxymethyl, Allyloxymethyl, Propargyloxymethyl, Ethoxymethoxymethyl, Methoxyethoxymethyl, Methylmercaptomethyl, Methoxy, Ethoxy, Methylmercapto, Methylsulfonyloxymethyl, Methoxycarbonyl, Ethoxycarbonyl, Chlormethyl, Dichlormethyl, Bromethyl, Pyrazol-1-yl-methyl, Imidazol-1-yl-methyl, 1,2,4-Triazol-1-yl-methyl, Benzyl und Tetrahydropyran-2-yl-oxymethyl steht ; sowie für Cyclopropyl, Cyclobutyl, Cyclopentyl, Cyclohexyl, Cyclobutenyl, Cyclopentenyl, Cyclohexenyl oder Cycloheptenyl steht, die einfach oder mehrfach, gleich oder verschieden substituiert sein können durch Methyl, Ethyl, Vinyl, Allyl, Fluor, Chlor, Chlormethyl, Trifluormethyl, Dichlorvinyl, Dibromvinyl, Methoxy, Trimethylen, Isobutylen.

Im einzelnen seien außer den bei den Herstellungsbeispielen genannten Verbindungen die folgenden Verbindungen der allgemeinen Formel (I) genannt :

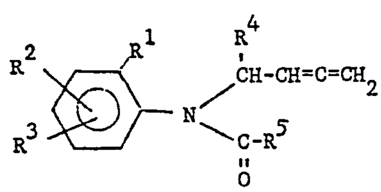

(I)

| $R^1$ | $R^2$ | $R^3$ | $R^4$ | $R^5$ |
|---|---|---|---|---|
| $CH_3$ | $6-CH_3$ | H | H | 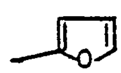 |
| $CH_3$ | $6-CH_3$ | H | H | $-CH_2-$ 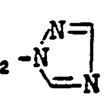 |

(Fortsetzung)

| $R^1$ | $R^2$ | $R^3$ | $R^4$ | $R^5$ |
|---|---|---|---|---|
| $CH_3$ | $6-CH_3$ | H | H | $-CH_2-$ 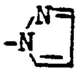 |
| $CH_3$ | $6-CH_3$ | H | H | $-CH_2-O-CH_2-O-C_2H_5$ |
| $CH_3$ | $6-CH_3$ | H | H | $-CH_2-O-CH_2CH_2-O-CH_3$ |
| $CH_3$ | $6-CH_3$ | H | H | $-CH_2-O-$ 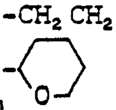 |
| $CH_3$ | $6-CH_3$ | H | H | 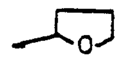 |
| $CH_3$ | $6-CH_3$ | H | H | 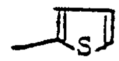 |
| $CH_3$ | $6-CH_3$ | H | H | $-CH_2-S-CH_3$ |
| $CH_3$ | $6-CH_3$ | H | H | $-CH_2Cl$ |
| $CH_3$ | $6-CH_3$ | H | H | $-CHCl_2$ |
| $CH_3$ | $6-CH_3$ | H | H | $-CCOCH_3$ |
| $CH_3$ | $6-CH_3$ | H | H | $-CH_2OSO_2CH_3$ |
| $CH_3$ | $6-CH_3$ | H | H | 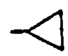 |
| $CH_3$ | $6-CH_3$ | H | H | 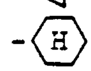 |
| $CH_3$ | $6-CH_3$ | H | H | 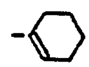 |
| $CH_3$ | $6-CH_3$ | H | H | 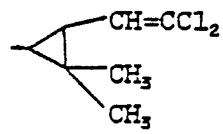 |
| $CH_3$ | $6-CH_3$ | H | H | 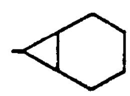 |
| $CH_3$ | $6-CH_3$ | H | H | $-CH_2-$ 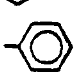 |
| $CH_3$ | $6-CH_3$ | H | $CH_3$ | 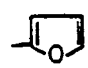 |
| $CH_3$ | $6-CH_3$ | H | $CH_3$ | $-CH_2-O-CH_2-O-C_2H_5$ |
| $CH_3$ | $6-CH_3$ | H | $CH_3$ | $-CH_2-O-$ 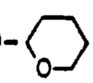 |
| $CH_3$ | $6-CH_3$ | H | $CH_3$ | 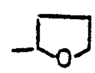 |
| $CH_3$ | $6-CH_3$ | H | $CH_3$ | 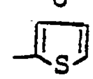 |
| $CH_3$ | $6-CH_3$ | H | $CH_3$ | $-CH_2-S-CH_3$ |
| $CH_3$ | $6-CH_3$ | H | $CH_3$ | $-CHCl_2$ |
| $CH_3$ | $6-CH_3$ | H | $CH_3$ | $-COOCH_3$ |
| $CH_3$ | $6-CH_3$ | H | $CH_3$ | 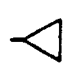 |
| $CH_3$ | $6-CH_3$ | H | $CH_3$ | 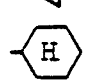 |
| $CH_3$ | $6-CH_3$ | H | $CH_3$ | 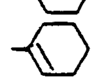 |

(Fortsetzung)

| $R^1$ | $R^2$ | $R^3$ | $R^4$ | $R^5$ |
|---|---|---|---|---|
| $CH_3$ | $6-CH_3$ | H | $CH_3$ | 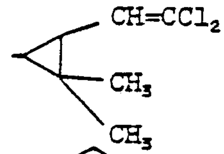 |
| $CH_3$ | $6-CH_3$ | H | $CH_3$ | 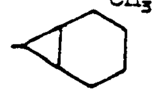 |
| $CH_3$ | $6-C_2H_5$ | H | H | 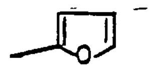 |
| $CH_3$ | $6-C_2H_5$ | H | H | $-CH_2-$ 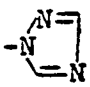 |
| $CH_3$ | $6-C_2H_5$ | H | H | $-CH_2-$ 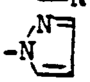 |
| $CH_3$ | $6-C_2H_5$ | H | H | $-CH_2-O-CH_2-O-C_2H_5$ |
| $CH_3$ | $6-C_2H_5$ | H | H | $-CH_2-O-CH_2CH_2-O-CH_3$ |
| $CH_3$ | $6-C_2H_5$ | H | H | $-CH_2-O-$ 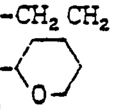 |
| $CH_3$ | $6-C_2H_5$ | H | H | 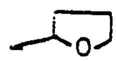 |
| $CH_3$ | $6-C_2H_5$ | H | H | 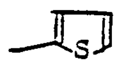 |
| $CH_3$ | $6-C_2H_5$ | H | H | $-CH_2-S-CH_3$ |
| $CH_3$ | $6-C_2H_5$ | H | H | $-CH_2Cl$ |
| $CH_3$ | $6-C_2H_5$ | H | H | $-CHCl_2$ |
| $CH_3$ | $6-C_2H_5$ | H | H | $-COOCH_3$ |
| $CH_3$ | $6-C_2H_5$ | H | H | $-CH_2OSO_2CH_3$ |
| $CH_3$ | $6-C_2H_5$ | H | H | 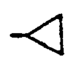 |
| $CH_3$ | $6-C_2H_5$ | H | H | 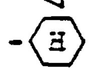 |
| $CH_3$ | $6-C_2H_5$ | H | H | 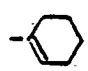 |
| $CH_3$ | $6-C_2H_5$ | H | H | 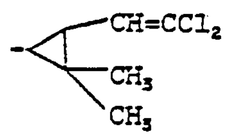 |
| $CH_3$ | $6-C_2H_5$ | H | H | 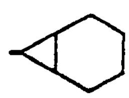 |
| $CH_3$ | $6-C_2H_5$ | H | H | $-CH_2-$ 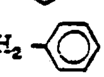 |
| $CH_3$ | 6-Cl | H | $CH_3$ | 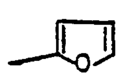 |
| $CH_3$ | 6-Cl | H | $CH_3$ | $-CH_2-$ 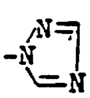 |
| $CH_3$ | 6-Cl | H | $CH_3$ | $-CH_2-$ 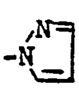 |

(Fortsetzung)

| $R^1$ | $R^2$ | $R^3$ | $R^4$ | $R^5$ |
|---|---|---|---|---|
| $CH_3$ | 6-Cl | H | $CH_3$ | $-CH_2-O-CH_2-O-C_2H_5$ |
| $CH_3$ | 6-Cl | H | $CH_3$ | $-CH_2-O-CH_2CH_2-O-CH_3$ |
| $CH_3$ | 6-Cl | H | $CH_3$ | 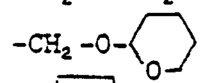 |
| $CH_3$ | 6-Cl | H | $CH_3$ | 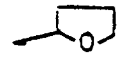 |
| $CH_3$ | 6-Cl | H | $CH_3$ | 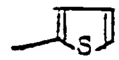 |
| $CH_3$ | 6-Cl | H | $CH_3$ | $-CH_2-S-CH_3$ |
| $CH_3$ | 6-Cl | H | $CH_3$ | $-CH_2Cl$ |
| $CH_3$ | 6-Cl | H | $CH_3$ | $-CHCl_2$ |
| $CH_3$ | 6-Cl | H | $CH_3$ | $-COOCH_3$ |
| $CH_3$ | 6-Cl | H | $CH_3$ | $-CH_2OSO_2CH_3$ |
| $CH_3$ | 6-Cl | H | $CH_3$ | 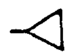 |
| $CH_3$ | 6-Cl | H | $CH_3$ | 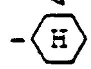 |
| $CH_3$ | 6-Cl | H | $CH_3$ | 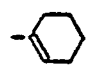 |
| $CH_3$ | 6-Cl | H | $CH_3$ | 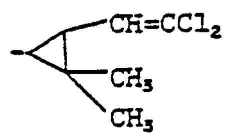 |
| $CH_3$ | 6-Cl | H | $CH_3$ | 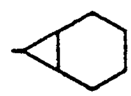 |
| $CH_3$ | 6-Cl | H | $CH_3$ | 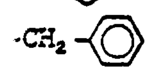 |

Verwendet man beispielsweise 2,6-Dimethyl-N-(1-methylpropargyl)-methoxyacetanilid, Paraformaldehyd und Di-isopropylamin als Ausgangsstoffe und Kupfer-I-bromid als Katalysator, so kann der Reaktionsablauf durch das folgende Formelschema wiedergegeben werden :

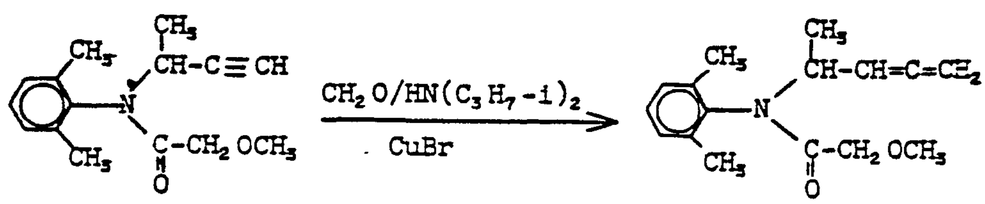


Die bei der Durchführung des erfindungsgemäßen Verfahrens als Ausgangsstoffe benötigten N-Propargyl-acetanilide sind durch die Formel (II) allgemein definiert. In dieser Formel stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) vorzugsweise für diese Substituenten genannt wurden.

Die N-Propargylacetanilide der Formel (I) sind weitgehend bekannt (vergleiche EP 0 005 591 und DE-OS 28 47 287 ; teilweise sind sie Gegenstand einer eigenen älteren Anmeldung, die noch nicht veröffentlicht ist (vergleiche die deutsche Patentanmeldung P 29 31 640 vom 3.8. 1979). Sie können nach den dort beschriebenen Verfahren erhalten werden, indem man z. B.

a) N-Propargylaniline der Formel

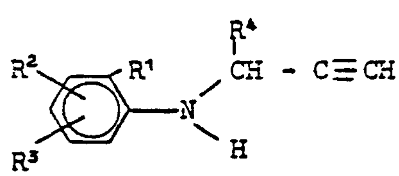 (III)

in welcher

$R^1$ bis $R^4$ die oben angegebene Bedeutung haben, mit Säurechloriden oder -bromiden bzw. -anhydriden der Formeln

$$R^5—CO—Cl(Br) \qquad (IVa)$$

bzw.

$$(R^5—CO)_2O \qquad (IVb)$$

in Gegenwart eines inerten organischen Lösungsmittels, wie beispielsweise Tetrahydrofuran, und gegebenenfalls in Gegenwart einer Base als Säurebinder, wie beispielsweise Pyridin, bei Temperaturen zwischen 20 und 100 °C umsetzt, oder

b) Anilide der Formel

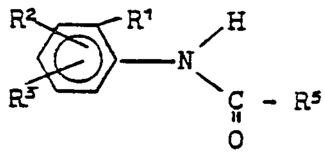

(V)

in welcher

$R^1$, $R^2$, $R^3$ und $R^5$ die oben angegebene Bedeutung haben, mit Propargylhalogeniden der Formel

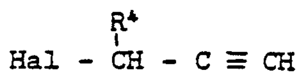

(VI)

in welcher

$R^4$ die oben angegebene Bedeutung hat und

Hal für Chlor oder Brom steht, in Gegenwart eines Säurebindemittels, wie beispielsweise Kaliumcarbonat und in Gegenwart eines inerten organischen Lösungsmittels, wie beispielsweise Ether, oder in Gegenwart eines wässrig-organischen Zweiphasensystems in Gegenwart eines Phasentransferkatalysators bei Temperaturen zwischen − 20 und + 80 °C umsetzt.

Die Propargylaniline der Formel (III) sind bekannt (vergleiche die US-Patentschriften 3 535 577 und 4 001 325, EP 0 005 591 sowie DE-OS 28 47 287).

Die Säurechloride, -bromide und -anhydride der Formeln (IVa) und (IVb) sind allgemein bekannte Verbindungen der organischen Chemie.

Die Anilide der Formel (V) sind ebenfalls bekannt (vergleiche DE-OS 28 47 287), bzw. können sie in allgemein bekannter Art und Weise erhalten werden, indem man entsprechende Aniline mit einem Säurechlorid oder -bromid bzw. -anhydrid der Formeln (IVa) bzw. (IVb) oder mit der Säure selbst gemäß den Bedingungen des Verfahrens (a) umsetzt.

Die Propargylhalogenide der Formel (VI) sind allgemein bekannte Verbindungen der organischen Chemie.

Die außerdem für das erfindungsgemäße Verfahren als Ausgangsstoffe benötigten Verbindungen Formaldehyd (in Form von Paraformaldehyd oder Trioxan) und Diisopropylamin sind allgemein bekannte Verbindungen der organischen Chemie.

Als Verdünnungsmittel kommen für die erfindungsgemäße Reaktion vorzugsweise inerte organischen Lösungsmittel infrage. Hierzu gehören vorzugsweise Ether, wie Tetrahydrofuran oder Dioxan.

Als Katalysator kommen für die erfindungsgemäße Reaktion vorzugsweise Kupfer-I-halogenide, wie Kupfer-I-bromid oder -chlorid infrage.

Bei der Durchführung des erfindungsgemäßen Verfahrens setzt man vorzugsweise auf 1 Mol der Verbindungen der Formel (II) 1 bis 2 Mol Formaldehyd, 1 bis 1,5 Mol Diisopropylamin und 0,1 bis 0,5 Mol Cu-I-halogenid ein. Die Isolierung der Endprodukte erfolgt nach üblichen Methoden (vergleiche auch die Angaben in J. Chem. Soc. Chem. Comm. 1979, 859; Tetrahedron Letters 21, 929 (1980) sowie die Herstellungsbeispiele).

Die Reaktionstemperaturen können bei der Durchführung des erfindungsgemäßen Verfahrens in einem größeren Bereich variiert werden. Im allgemeinen arbeitet man zwischen 60 und 120 °C, vorzugsweise bei der Siedetemperatur des verwendeten Lösungsmittels.

Zur Herstellung von physiologisch verträglichen Säureadditionssalzen der Verbindungen der Formel (I) kommen vorzugsweise folgende Säuren infrage: Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten : Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z. B. gegen den Erreger der Kraut- und Braunfäule der Tomate und Kartoffel (Phytophthora infestans) eingesetzt werden. Besonders hervorzuheben ist, daß die erfindungsgemäßen Wirkstoffe nicht nur eine protektive, sondern auch eine kurativ-eradikative Wirkung entfalten. Außerdem besitzen sie systemische Eigenschaften. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wessentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Füssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid ; als feste Trägerstoffe kommen in Frage : z. B. natürliche Gesteinsmehlem wie Kaoline, Tonerden, Talkum, Kreide, Quarz, Attapulgit, Montmorillonit oder Diatomeerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürlich und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff,

vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als soche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 1 bis 0,1 Gew.-%, vorzugsweise von 0,000 1 bis 0,02 %, am Wirkungsort erforderlich.

## Herstellungsbeispiele

### Beispiel 1

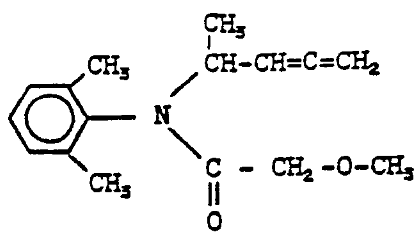

Eine Lösung von 98,1 g (0,4 Mol) 2',6'-Dimethyl-2-methoxy-N-(1-methylpropargyl)-acetanilid in 800 ml Dioxan wird mit 19,2 g (0,64 Mol) Paraformaldehyd, 48,5 g (0,48 Mol) Diisopropylamin und 18,9 g (0,132 Mol) Kupfer-(I)-bromid versetzt und 6 Stunden unter Rückfluß erhitzt. Die Reaktionslösung wird filtriert und eingedampft. Den Rückstand verteilt man zwischen je 400 ml Essigester und 1 molarer Citronensäure, wäscht die organische Phase mit 400 ml gesättigter Natriumhydrogencarbonat-Lösung und Wasser, trocknet über Natriumsulfat und dampft ein. Der dunkelbraune Rückstand (57,9 g) wird an einer Kieselgel-Säule (6 × 100 cm/Korngröße 0,063-0,2 mm) chromatographiert, wobei mit je 6 l 10 % und 20 % sowie 3,5 l 30 % Essigester in Toluol eluiert wird. Man erhält so 37,7 g (36,4 % der Theorie) 2',6'-Dimethyl-2-methoxy-N-(1-methyl-2,3-butadienyl)-acetanilid als gelbes Oel : $n_D^{25}$ = 1,531 7.

In analoger Weise werden die nachfolgenden Verbindungen der allgemeinen Formel

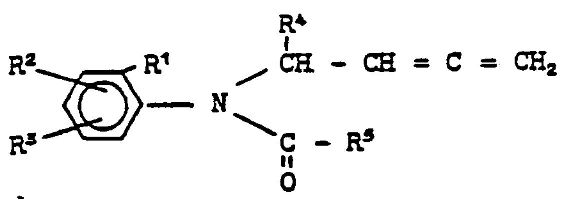 (I)

erhalten :

| Bsp. Nr. | $R^1$ | $R^2$ | $R^3$ | $R^4$ | $R^5$ | Physik. Konst. |
|---|---|---|---|---|---|---|
| 2 | $CH_3$ | 6-$CH_3$ | H | $CH_3$ | $-CH_2-OSO_2-CH_3$ | Oel, $n_D^{25}$=1,5326 |
| 3 | $CH_3$ | 6-$CH_3$ | H | H | $-CH_2-O-CH_3$ | $n_D^{20}$=1,5411 |
| 4 | $CH_3$ | 6-$CH_3$ | H | H | $-CH=CH-CH_3$ | Oel, viskos, IR: 1950 $cm^{-1}$, 1655 $cm^{-1}$ |
| 5 | $CH_3$ | 6-$CH_3$ | H | $CH_3$ | 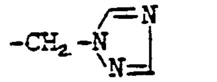 | Oel, $n_D^{25}$ = 1,5488 |

(Fortsetzung)

| Bsp. Nr. | $R^1$ | $R^2$ | $R^3$ | $R^4$ | $R^5$ | Physik. Konst. |
|---|---|---|---|---|---|---|
| 6 | $CH_3$ | 6-$CH_3$ | H | $CH_3$ | $-CH_2Cl$ | Oel, $n_D^{25}$ = 1,5483 |
| 7 | $CH_3$ | 6-$CH_3$ | H | $CH_3$ | $-CH_2-$ 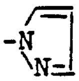 | Oel, $n_D^{25}$ = 1,5570 |
| 8 | $CH_3$ | 6-$CH_3$ | H | $CH_3$ | $-CH_2-$ 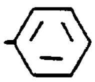 | Oel, $n_D^{25}$ = 1,5654 |
| 9 | $CH_3$ | 6-$CH_3$ | H | $CH_3$ | $-CH_2OCH_2CH_2-O-CH_3$ | Oel, $n_D^{25}$ = 1,5208 |
| 10 | $CH_3$ | 6-$CH_3$ | H | H | $-CH_2-O-CH_2-CH=C=CH_2$ | Oel, $n_D^{20}$ = 1,5522 |
| 11 | $CH_3$ | 6-$CH_3$ | H | $CH_3$ | $-CH_2-$ 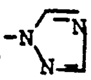 | Fp. 157-58°C (x 1/2 $CuCl_2$) |
| 12 | $CH_3$ | 6-$CH_3$ | H | $CH_3$ | $-CH_2-$ 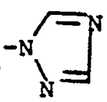 | Fp. 198-200°C (x 1/2 $ZnCl_2$) |
| 13 | $CH_3$ | 6-$CH_3$ | H | $CH_3$ | $-CH_2-$ 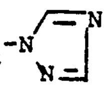 | Fp. 224-25°C (x 1/2 $MnCl_2$) |
| 14 | $CH_3$ | 6-$CH_3$ | H | $CH_3$ | 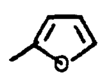 | Fp. 53-54°C |
| 15 | $CH_3$ | 6-$CH_3$ | H | $CH_3$ | $-COOCH_3$ | $n_D^{20}$ = 1,5242 |
| 16 | $CH_3$ | 6-$CH_3$ | H | $CH_3$ | $-CH_2-S-CH_3$ | $n_D^{20}$ = 1,5554 |
| 17 | $CH_3$ | 6-$CH_3$ | H | $CH_3$ | $-CHCl_2$ | zähes Öl |
| 18 | $CH_3$ | 6-$CH_3$ | H | $CH_3$ | 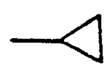 | $n_D^{20}$ = 1,5389 |
| 19 | $CH_3$ | 6-$CH_3$ | H | $CH_3$ | $-CH=CH-CH_3$ | $n_D^{20}$ = 1,5457 |
| 20 | $CH_3$ | 6-$CH_3$ | H | $CH_3$ | $-CH_2Br$ | $n_D^{20}$ = 1,5477 |
| 21 | Cl | 6-$CH_3$ | H | $CH_3$ | $-CH_2-O-CH_3$ | $n_D^{20}$ = 1,5382 |

Anwendungsbeispiel

In dem nachfolgenden Beispiel wird die nachstehend angegebene Verbindung als Vergleichssubstanz eingesetzt :

(A) = 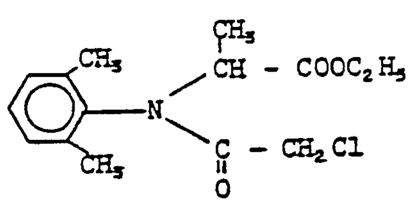

N-Chloracetyl-N-(2,6-dimethylphenyl)-alanin-ethylester.

Beispiel A

Phytophthora-Test (Tomaten)/Protektiv

Lösungsmittel : 4,7 Gewichtsteile Aceton
Emulgator : 0,3 Gewichtsteile Alkyl-aryl-polyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20 °C und einer relativen Luftfeuchtigkeit von 70 % im Gewächshaus. Anschließend werden die Tomatenpflanzen mit einer wässrigen Sporensuspension von Phytophthora infestans inokuliert. Die Pflanzen werden in eine Feuchtkammer mit einer 100 %-igen Luftfeuchtigkeit und einer Temperatur von 18 bis 20 °C gebracht.

Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

Die erfindungsgemäßen Wirkstoffe 1, 2, 4 und 5 z. B. zeigen in diesem test eine bessere Wirkung als die aus dem Stand der Technik bekannte Substanz (A).

**Ansprüche**

1. N-Allenylalkyl-acetanilide der allgemeinen Formel

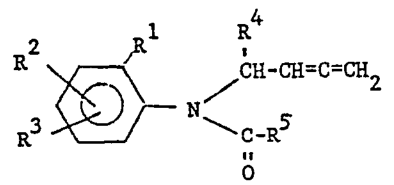

(I)

in welcher

$R^1$ für Wasserstoff, Alkyl mit 1 bis 4 Kohlenstoffatomen oder Halogen steht,

$R^2$ für Wasserstoff oder Alkyl mit 1 bis 4 Kohlenstoffstoffatomen steht,

$R^3$ für Wasserstoff oder Alkyl mit 1 bis 4 Kohlenstoffatomen steht,

$R^4$ für Wasserstoff oder Alkyl mit 1 bis 4 Kohlenstoffatomen steht,

$R^5$ für Furyl, Tetrahydrofuryl, Thiophenyl, Tetrahydrothiophenyl ; gegebenenfalls durch Methyl oder Ethyl substituiertes Isoxazolyl ; gegebenenfalls durch Cyano oder Thiocyanato substituiertes Alkyl mit 1 bis 4 Kohlenstoffatomen, Alkenyl und Alkinyl mit jeweils 2 bis 4 Kohlenstoffatomen, gegebenenfalls substituiertes Cycloalkyl mit 3 bis 7 Kohlenstoffatomen oder Cycloalkenyl mit jeweils 4 bis 7 Kohlenstoffatomen, wobei als Substituenten genannt seien : Alkyl mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, Halogenalkenyl mit 2 bis 4 Kohlenstoff- und bis zu 5 gleichen oder verschiedenen Halogenatomen, Halogen, Alkoxy mit 1 bis 4 Kohlenstoffatomen sowie ein zwei- bis fünfgliedrige Methylenbrücke ; ferner für Halogenalkyl mit 1 oder 2 Kohlenstoffatomen und 1 oder 2 Halogenatomen ; sowie die Gruppierungen $-CH_2-Az$, $CH_2OR^6$, $-CH_2-SR^6$, $-OR^6$, $-SR^6$, $-CH_2-OSO_2R^6$, $-COOR^6$, $-CH_2-Ar$ und

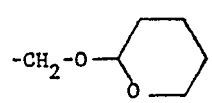

steht, wobei

$R_6$ für gegebenenfalls durch Halogen, Cyano und Thiocyanato substituiertes Alkyl mit 1 bis 4 Kohlenstoffatomen, Alkenyl mit 2 bis 4 Kohlenstoffatomen, Alkinyl mit 2 bis 4 Kohlenstoffatomen und Alkoxyalkyl mit 1 bis 4 Kohlenstoffatomen in jedem Alkylteil steht,

Az für Pyrazol-1-yl, 1,2,4-Triazol-1-yl und Imidazo-1-yl steht,

Ar für gegebenenfalls substituiertes Phenyl steht, wobei als Substituenten genannt seien : Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, Nitro, Cyano sowie gegebenenfalls durch Halogen und Alkyl mit 1 bis 4 Kohlenstoffatomen substituiertes Phenyl, sowie, sofern $R^5$ für die Bedeutung $-CH_2-Az$ steht, deren physiologisch verträglichen Säureadditions-Salze und deren Metallsalz-Komplexe.

2. N-Allenylalkyl-acetanilide der allgemeinen Formel I in Anspruch 1, wobei

$R^1$, $R^2$ und $R^3$ für Wasserstoff, Methyl, Ethyl, Isopropyl, sek.-Butyl und tert.-Butyl stehen und $R^1$ außerdem noch für Chlor oder Brom steht,

$R^4$ für Wasserstoff, Methyl oder Ethyl steht, und

$R^5$ für 2-Furyl, 2-Thienyl, 2-Tetrahydrofuryl, 5-Methyl-isoxazol-3-yl, Methoxymethyl, Ethoxymethyl, Allyloxymethyl, Propargyloxymethyl, Ethoxymethoxymethyl, Methoxyethoxymethyl, Methylmercaptomethyl, Methoxy, Ethoxy, Methymercapto, Methylsulfonyloxymethyl, Methoxycarbonyl, Ethoxycarbonyl, Chlormethyl, Dichlormethyl, Pyrazol-1-yl-methyl, Imidazol-1-yl-methyl, 1,2,4-Triazol-1-yl-methyl, Benzyl und Tetrahydropyran-2-yl-oxymethyl steht, sowie für Cyclopropyl, Cyclobutyl, Cyclopentyl, Cyclohexyl, Cyclobutenyl, Cyclopentenyl, Cyclohexenyl oder Cycloheptenyl steht, die einfach oder mehrfach, gleich oder verschieden substituiert sein können durch Methyl, Ethyl, Vinyl, Allyl, Fluor, Chlor, Chlormethyl, Trifluormethyl, Dichlorvinyl, Dibromvinyl, Methoxy, Trimethylen und Isobutylen.

3. Verfahren zur Herstellung von N-Allenylalkyl-acetaniliden der allgemeinen Formel

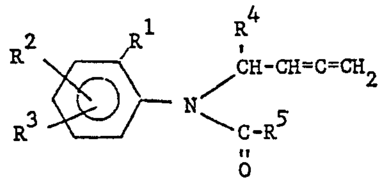

(I)

in welcher

$R^1$ für Wasserstoff, Alkyl mit 1 bis 4 Kohlenstoffatomen oder Halogen steht,

$R^2$ für Wasserstoff oder Alkyl mit 1 bis 4 Kohlenstoffatomen steht,

$R^3$ für Wasserstoff oder Alkyl mit 1 bis 4 Kohlenstoffatomen steht,

$R^4$ für Wasserstoff oder Alkyl mit 1 bis 4 Kohlenstoffatomen steht,

$R^5$ für Furyl, Tetrahydrofuryl, Thiophenyl, Tetrahydrothiophenyl ; gegebenenfalls durch Methyl oder Ethyl substituiertes Isoxazolyl ; gegebenenfalls durch Cyano oder Thiocyanato substituiertes Alkyl mit 1 bis 4 Kohlenstoffatomen, Alkenyl und Alkinyl mit jeweils 2 bis 4 Kohlenstoffatomen ; gegebenenfalls substituiertes Cycloalkyl mit 3 bis 7 Kohlenstoffatomen oder Cycloalkenyl mit 4 bis 7 Kohlenstoffatomen wobei die Substituenten genannt seien ; Alkyl mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, Halogenalkyl mit 2 bis 4 Kohlenstoff- und bis zu 5 gleichen oder verschiedenen Halogenatomen, Halogen, Alkoxy mit 1 bis 4 Kohlenstoffatomen sowie eine zwei- bis fünfgliedrige Methylenbrücke ; ferner für Halogenalkyl mit 1 oder 2 Kohlenstoffatomen und 1 oder 2 Halogenatomen ; sowie die Gruppierungen $-CH_2-Az$, $-CH_2-OR^6$, $-CH_2SR^6$, $-OR^6$, $-SR^6$, $-CH_2-OSO_2R^6$, $-COOR^6$, $-CH_2-Ar$ und

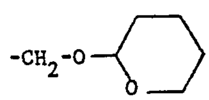

steht, wobei

$R^6$ für gegebenenfalls durch Halogen, Cyano und Thiocyanato substituiertes Alkyl mit 1 bis 4 Kohlenstoffatomen, Alkenyl mit 2 bis 4 Kohlenstoffatomen, Alkinyl mit 2 bis 4 Kohlenstoffatomen und Alkoxyalkyl mit 1 bis 4 Kohlenstoffatomen in jedem Alkylteil steht,

Az für Pyrazol-1-yl, 1,2,4-Triazol-1-yl und Imidazol-1-yl steht, und

Ar für gegebenenfalls substituiertes Phenyl steht, wobei als Substituenten genannt seien : Halogen, Alkyl, Alkoxy und Alkylthio mit jeweils 1 bis 4 Kohlenstoffatomen, Halogenalkyl, Halogenalkoxy und Halogenalkylthio mit jeweils 1 oder 2 Kohlenstoff- und 1 bis 5 gleichen oder verschiedenen Halogenatomen, Nitro, Cyano sowie gegebenenfalls durch Halogen und Alkyl mit 1 bis 4 Kohlenstoffatomen substituiertes Phenyl sowie, sofern $R^5$ für die Bedeutung $-CH_2-Az$ steht, von deren physiologisch verträglichen Säureadditions-Salze und Metallsalz-Komplexe, dadurch gekennzeichnet, daß man N-Propargyl-acetanilide der Formel

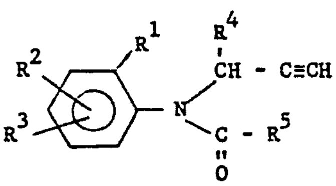

(II)

in welcher

$R^1$ bis $R^5$ die oben angegebene Bedeutung haben, in Gegenwart eines Katalysators mit Formaldehyd

und Diisopropylamin umsetzt, und an die so erhaltenen Verbindungen der Formel (I) für den Fall, daß $R^5$ für $—CH_2—Az$ steht, noch gegebenenfalls eine Säure oder ein Metallsalz addiert.

4. Fungizide Mittel, gekennzeichnet durch einen Gehalt an mindestens einem N-Allenylalkyl-acetanilid der Formel I.

5. Verfahren zur Bekämpfung von Pilzen, dadurch gekennzeichnet, daß man N-Allenylalkylacetanilide der Formel I auf Pilze oder ihren Lebensraum einwirken läßt.

6. Verwendung von N-Allenylalkyl-acetaniliden der Formel I zur Bekämpfung von Pilzen.

7. Verfahren zur Herstellung von Fungiziden Mitteln, dadurch gekennzeichnet, daß man N-Allenylalkylacetanilide der Formel I mit Streckmitteln und/oder oberflächenaktiven Mitteln vermischt.

**Claims**

1. N-Allenylalkyl-acetanilides of the general formula

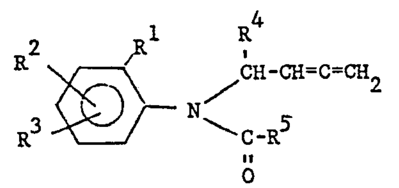

(I)

in which

$R^1$ represents hydrogen, alkyl with 1 to 4 carbon atoms or halogen,

$R^2$ represents hydrogen or alkyl with 1 to 4 carbon atoms,

$R^3$ represents hydrogen or alkyl with 1 to 4 carbon atoms,

$R^4$ represents hydrogen or alkyl with 1 to 4 carbon atoms,

$R^5$ represents furyl, tetrahydrofuryl, thiophenyl or tetrahydrothiophenyl ; isoxazolyl which is optionally substituted by methyl or ethyl ; alkyl with 1 to 4 carbon atoms, alkenyl or alkinyl with in each case 2 to 4 carbon atoms, each optionally substituted by cyano or thiocyanato, optionally substituted cycloalkyl with 3 to 7 carbon atoms or cycloalkenyl with in each case 4 to 7 carbon atoms, substituents which may be mentioned being : alkyl with 1 to 4 carbon atoms, alkenyl with 2 to 4 carbon atoms, halogenoalkyl with 1 to 4 carbon atoms and up to 5 identical or different halogen atoms, halogenoalkenyl with 2 to 4 carbon atoms and up to 5 identical or different halogen atoms, halogen, alkoxy with 1 to 4 carbon atoms and a two-membered to five-membered methylene bridge ; or furthermore represents halogenoalkyl with 1 or 2 carbon atoms and 1 or 2 halogen atoms ; or represents the groupings $—CH_2—Az$, $—CH_2OR^6$, $—CH_2—SR^6$, $—OR^6$, $—SR^6$, $—CH_2—OSO_2R^6$, $—COOR^6$, $—CH_2—Ar$ or

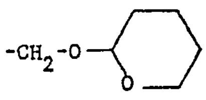

wherein

$R^6$ represents alkyl with 1 to 4 carbon atoms, alkenyl with 2 to 4 carbon atoms, alkinyl with 2 to 4 carbon atoms or alkoxyalkyl with 1 to 4 carbon atoms in each alkyl part, each optionally substituted by halogen, cyano or thiocyanato,

Az represents pyrazol-1-yl, 1,2,4-triazol-1-yl or imidazol-1-yl,

Ar represents optionally substituted phenyl, substituents which may be mentioned being : halogen, alkyl, alkoxy and alkylthio with in each case 1 to 4 carbon atoms, halogenoalkyl, halogenoalkoxy and halogenoalkylthio with in each case 1 to 2 carbon atoms and 1 to 5 identical or different halogen atoms, nitro, cyano and phenyl which is optionally substituted by halogen and alkyl with 1 to 4 carbon atoms, and, if $R^5$ represents $—CH_2—Az$, physiologically acceptable acid addition salts thereof and metal salt complexes thereof.

2. N-Allenylalkyl-acetanilides of the general formula I in Claim 1, wherein

$R^1$, $R^2$ and $R^3$ represent hydrogen, methyl, ethyl, isopropyl, sec.-butyl or tert.-butyl and

$R^1$ also represents chlorine or bromine,

$R^4$ represents hydrogen, methyl or ethyl and

$R^5$ represents 2-furyl, 2-thienyl, 2-tetrahydrofuryl, 5-methyl-isoxazol-3-yl, methoxymethyl, ethoxymethyl, allyloxymethyl, propargyloxymethyl, ethoxymethoxymethyl, methoxyethoxymethyl, methylmercaptomethyl, methoxy, ethoxy, methylmercapto, methylsulphonyloxymethyl, methoxycarbonyl, ethoxycarbonyl, chloromethyl, dichloromethyl, pyrazol-1-yl-methyl, imidazol-1-yl-methyl, 1,2,4-triazol-1-yl-methyl, benzyl or tetrahydropyran-2-yl-oxymethyl, or represents cyclopropyl, cyclobutyl, cyclopentyl, cyclohexyl, cyclobutenyl, cyclopentenyl, cyclohexenyl or cycloheptenyl, which can be monosubstituted or polysubstituted by identical or different substituents from the group comprising methyl, ethyl, vinyl,

allyl, fluorine, chlorine, chloromethyl, trifluoromethyl, dichlorovinyl, dibromovinyl, methoxy, trimethylene and isobutylene.

3. Process for the preparation of N-allenylalkyl-acetanilides of the general formula

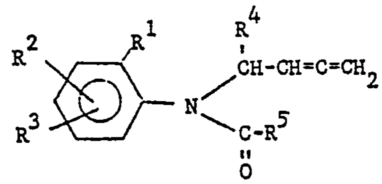

(I)

in which

$R^1$ represents hydrogen, alkyl with 1 to 4 carbon atoms or halogen,

$R^2$ represents hydrogen or alkyl with 1 to 4 carbon atoms,

$R^3$ represents hydrogen or alkyl with 1 to 4 carbon atoms,

$R^4$ represents hydrogen or alkyl with 1 to 4 carbon atoms,

$R^5$ represents furyl, tetrahydrofuryl, thiophenyl or tetrahydrothiophenyl ; isoxazolyl which is optionally substituted by methyl or ethyl ; alkyl with 1 to 4 carbon atoms, alkenyl or alkinyl with in each case 2 to 4 carbon atoms, each optionally substituted by cyano or thiocyanato ; optionally substituted cycloalkyl with 3 to 7 carbon atoms or cycloalkenyl with 4 to 7 carbon atoms, substituents which may be mentioned being : alkyl with 1 to 4 carbon atoms, alkenyl with 2 to 4 carbon atoms, halogenoalkyl with 1 to 4 carbon atoms and up to 5 identical or different halogen atoms, halogenoalkenyl with 2 to 4 carbon atoms and up to 5 identical or different halogen atoms, halogen, alkoxy with 1 to 4 carbon atoms and a two-membered to five-membered methylene bridge ; or furthermore represents halogenoalkyl with 1 or 2 carbon atoms and 1 or 2 halogen atoms ; or represents the groupings $—CH_2—Az$, $—CH_2OR^6$, $—CH_2—SR^6$, $—OR^6$, $—SR^6$, $—CH_2—OSO_2R^6$, $—COOR^6$, $—CH_2—Ar$ or

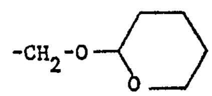

wherein

$R^6$ represents alkyl with 1 to 4 carbon atoms, alkenyl with 2 to 4 carbon atoms, alkinyl with 2 to 4 carbon atoms or alkoxyalkyl with 1 to 4 carbon atoms in each alkyl part, each optionally substituted by halogen, cyano or thiocyanato,

Az represents pyrazol-1-yl, 1,2,4-triazol-1-yl or imidazol-1-yl, and

Ar represents optionally substituted phenyl, substituents which may be mentioned being : halogen, alkyl, alkoxy and alkylthio with in each case 1 to 4 carbon atoms, halogenoalkyl, halogenoalkoxy and halogenoalkylthio with in each case 1 or 2 carbon atoms and 1 to 5 identical or different halogen atoms, nitro, cyano and phenyl which is optionally substituted by halogen and alkyl with 1 to 4 carbon atoms, and, if $R^5$ represents $—CH_2—Az$, physiologically acceptable acid addition salts thereof and metal salt complexes thereof, characterised in that N-propargyl-acetanilides of the formula

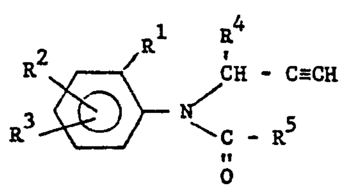

(II)

in which

$R^1$ to $R^5$ have the abovementioned meaning, are reacted with formaldehyde and diisopropylamine in the presence of a catalyst and, if appropriate, in the case where $R^5$ represents $—OH_2—Az$, an acid or a metal salt is then added on to the compounds of the formula (I) thus obtained.

4. Fungicidal agents, characterised in that they contain at least one N-allenylalkyl-acetanilide of the formula I.

5. Process for combating fungi, characterised in that N-allenylalkylacetanilides of the formula I are allowed to act on fungi or their environment.

6. Use of N-allenylalkyl-acetanilides of the formula I for combating fungi.

7. Process for the preparation of fungicidal agents, characterised in that N-allenylalkylacetanilides of the formula I are mixed with extenders and/or surface-active agents.

**Revendications**

1. N-allényIalkyl-acétanilides de formule générale

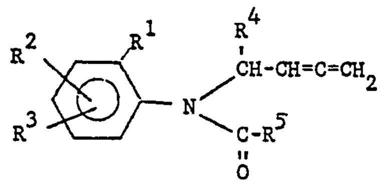

(I)

dans laquelle

$R^1$ représente l'hydrogène ou un halogène ou un groupe alkyle en $C_1$-$C_4$,

$R^2$ représente l'hydrogène ou un groupe alkyle en $C_1$-$C_4$,

$R^3$ représente l'hydrogène ou un groupe alkyle en $C_1$-$C_4$,

$R^4$ représente l'hydrogène ou un groupe alkyle en $C_1$-$C_4$,

$R^5$ représente un groupe furyle, tétrahydrofuryle, thiophényle, tétrahydrothiophényle ; isoxazolyle éventuellement substitué par méthyle ou éthyle ; alkyle en $C_1$-$C_4$, alcényle et alcynyle en $C_2$-$C_4$, éventuellement substitués par cyano ou thiocyanato ; cycloalkyle en $C_3$-$C_7$ ou cycloalcényle en $C_4$-$C_7$ éventuellement subsititué, les substituants pouvant être : alkyle en $C_1$-$C_4$, alcényle en $C_2$-$C_4$, halogénoalkyle en $C_1$-$C_4$ et ayant jusqu'à 5 atomes d'halogène identiques ou différents, halogénoalcényle en $C_2$-$C_4$ et ayant jusqu'à 5 atomes d'halogène identiques ou différents, halogène, alcoxy en $C_1$-$C_4$ ainsi qu'un pont méthylénique à 2-5 chaînons ; en outre halogénoalkyle en $C_1$ ou $C_2$ et ayant 1 ou 2 atomes d'halogène ; ainsi que les groupements $-CH_2-Az$, $-CH_2-OR^6$ ; $-CH_2-SR^6$ ; $-OR^6$ ; $-SR^6$ ; $-CH_2-OSO_2R^6$, $-COOR^6$, $-CH_2-Ar$ et

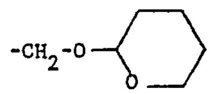

dans lesquels

$R^6$ représente un groupe alkyle en $C_1$-$C_4$ éventuellement substitué par halogène, cyano et thiocyanato, alcényle en $C_2$-$C_4$, alcynyle en $C_2$-$C_4$ et alcoxyalkyle où chaque reste alkyle est en $C_1$-$C_4$,

Az représente un reste pyrazole-1-yle, 1,2,4-triazole-1-yle et imidazole-1-yle, et

Ar représente un reste phényle éventuellement substitué, les substituants pouvant être les suivants : halogène, alkyle, alcoxy et alkylthio, chacun en $C_1$-$C_4$, halogénoalkyle, halogénoalcoxy et halogénoalkylthio, chacun en $C_1$-$C_2$, et ayant 1 à 5 atomes d'halogène identiques ou différents, nitro, cyano, ainsi que phényle éventuellement substitué par halogène et alkyle en $C_1$-$C_4$ ; ainsi que, lorsque $R^5$ est un reste $-CH_2Az$, leurs sels d'addition d'acides et leurs complexes de sels métalliques physiologiquement acceptables.

2. N-allénylalkyl-acétanilides substitués de formule générale (I) selon la revendication 1, dans lesquels

$R^1$, $R^2$ et $R^3$ représentent l'hydrogène ou un groupe méthyle, éthyle, isopropyle, sec-butyle et tert-butyle ;

$R^1$ représente en outre le chlore ou le brome ;

$R^4$ représente l'hydrogène ou un groupe méthyle ou éthyle ; et

$R^5$ représente un groupe 2-furyle, 2-thiényle, 2-tétrahydrofuryle, 5-méthyl-isoxazole-3-yle, méthoxyméthyle, éthoxyméthyle, allyloxyméthyle, propargyloxyméthyle, éthoxyméthoxyméthyle, méthoxyéthoxyméthyle, méthylmercaptométhyle, méthoxy, éthoxy, méthylmercapto, méthylsulfonyloxyméthyle, méthoxycarbonyle, éthoxycarbonyle, chlorométhyle, dichlorométhyle, bromométhyle, pyrazole-1-yl-méthyle, imidazole-1-yl-méthyle, 1,2,4-triazole-1-yl-méthyle, benzyle et tétrahydropyranne-2-yl-oxyméthyle ; ainsi qu'un groupe cyclopropyle, cyclobutyle, cyclopentyle, cyclohexyle, cyclobutényle, cyclopentényle, cyclohexényle ou cycloheptényle, qui peuvent porter un ou plusieurs substituants identiques ou différents choisis parmi méthyle, éthyle, vinyle, allyle, fluor, chlore, chlorométhyle, trifluorométhyle, dichlorovinyle, dibromovinyle, méthoxy, triméthylène, isobutylène.

3. Procédé pour la fabrication de N-allénylalkyl-acétanilides de formule générale

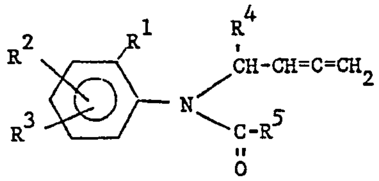

(I)

dans laquelle

$R^1$ représente l'hydrogène ou un halogène ou un groupe alkyle en $C_1$-$C_4$,

$R^2$ représente l'hydrogène ou un groupe alkyle en $C_1$-$C_4$,

$R^3$ représente l'hydrogène ou un groupe alkyle en $C_1$-$C_4$,

$R^4$ représente l'hydrogène ou un groupe alkyle en $C_1$-$C_4$,

$R^5$ représente un groupe furyle, tétrahydrofuryle, thiophényle, tétrahydrothiophényle ; isoxazolyle éventuellement substitué par méthyle ou éthyle ; alkyle en $C_1$-$C_4$, alcényle et alcynyle en $C_2$-$C_4$, éventuellement substitués par cyano ou thiocyanato ; cycloalkyle en $C_3$-$C_7$ ou cycloalcényle en $C_4$-$C_7$ éventuellement substitué, les substituants pouvant être : alkyle en $C_1$-$C_4$, alcényle en $C_2$-$C_4$, halogénoalkyle en $C_1$-$C_4$ et ayant jusqu'à 5 atomes d'halogène identiques ou différents, halogénoalcényle en $C_2$-$C_4$ et ayant jusqu'à 5 atomes d'halogène identiques ou différents, halogène, alcoxy en $C_1$-$C_4$ ainsi qu'un pont méthylénique à 2-5 chaînons ; en outre halogénoalkyle en $C_1$ ou $C_2$ et ayant 1 ou 2 atomes d'halogène ; ainsi que les groupements $-CH_2Az$, $-CH_2-OR^6$ ; $-CH_2-SR^6$ ; $-OR^6$ ; $-SR^6$ ; $-CH_2-OSO_2R^6$, $-COOR^6$, $-CH_2-Ar$ et

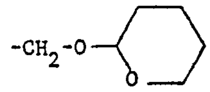

dans lesquels

$R^6$ représente un groupe alkyle en $C_1$-$C_4$ éventuellement substitué par halogène, cyano et thiocyanato, alcényle en $C_2$-$C_4$, alcynyle en $C_2$-$C_4$ et alcoxyalkyle où chaque reste alkyle est en $C_1$-$C_4$,

Az représente un reste pyrazole-1-yle, 1,2,4-triazole-1-yl et imidazole-1-yle, et

Ar représente un reste phényle éventuellement substitué, les substituants pouvant être les suivants : halogène, alkyle, alcoxy et alkylthio, chacun en $C_1$-$C_4$, halogénoalkyle, halogénoalcoxy et halogénoalkylthio, chacun en $C_1$-$C_2$, et ayant 1 à 5 atomes d'halogène identiques ou différents, nitro, cyano, ainsi que phényle éventuellement substitué par halogène et alkyle en $C_1$-$C_4$ ; ainsi que, lorsque $R^5$ est un reste $-CH_2Az$, leurs sels d'addition d'acides et leurs complexes de sels métalliques physiologiquement acceptables, caractérisé en ce que l'on fait réagir des N-propargyl-acétanilides de formule

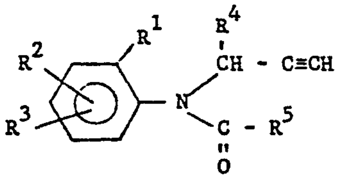

(II)

dans laquelle

$R^1$ à $R^5$ ont la signification indiquée ci-dessus, avec le formaldéhyde et la diisopropylamine en présence d'un catalyseur et, dans le cas où $R^5$ représente $-CH_2-Az$, on additionne encore éventuellement un acide ou un sel métallique sur les composés de formule (I) ainsi obtenus.

4. Agents fongicides, caractérisés en ce qu'ils contiennent au moins un N-allénylalkyl-acétanilide de formule I.

5. Procédé pour combattre les champignons, caractérisé en ce que l'on fait agir sur les champignons ou leur espace vital des N-allénylalkyl-acétanilides de formule I.

6. Utilisation des N-allénylalkyl-acétanilides de formule I pour combattre les champignons.

7. Procédé pour la fabrication d'agents fongicides, caractérisé en ce que l'on mélange des N-allénylalkyl-acétanilides de formule I avec des diluants et/ou des agents tensioactifs.